# EUROPEAN PATENT APPLICATION

(11) **EP 0 894 783 A1**
(43) Date of publication of application: **03.02.1999**
(21) Application number: 98112318.5
(22) Date of filing: 03.07.1998
(51) Int. Cl.: C07C 37/60, C07C 39/04

(54) **Process for the synthesis of phenol from benzene**

(30) Priority: 29.07.1997 IT MI971806
(71) Applicant: Enichem S.p.A., S. Donato Milanese (IT)
(72) Inventor: Tassinari, Roberto, 28100 Novara (IT); Bianchi, Daniele, 20020 Arese (Milano) (IT); Ungarelli, Raffaele, 28069 Trecate (Novara) (IT); Battistel, Ezio, 28063 Cameriano (Novara) (IT); d'Aloisio, Rino, 28067 Pernate (Novara) (IT)
(74) Representative: Gennari, Marco, Dr.

(57) **Abstract**

An improved process is described for the synthesis of phenol by the catalytic oxidation of benzene with hydrogen peroxide in the presence of titanium silicalite, wherein the improvement consists in the fact that the hydrogen peroxide is produced in situ by the reaction of oxygen, carbon monoxide and water in the presence of catalytic complexes of palladium with a nitrogenated ligand and a non-coordinating counter-ion. The process allows the production of phenol with a good yield and selectivity.

## Description

The present invention relates to an improved process for the synthesis of phenol by the catalytic oxidation of benzene with hydrogen peroxide in the presence of titanium silicalite, wherein the improvement consists in the fact that the hydrogen peroxide is produced in situ by the reaction of oxygen, carbon monoxide and water in the presence of catalytic complexes of palladium with a nitrogenated ligand and a non-coordinating counter-ion.

Phenol is a compound which is useful in the preparation of synthetic resins, insecticides and antioxidants.

The preparation of phenol by the direct oxidation of benzene with hydrogen peroxide in the presence of zeolites as catalysts, is known in the art (A. Thangaraj et al., J. Applied Catalysis, **57**, 1990).

Other processes are also known for the synthesis of phenol by the direct oxidation of benzene with hydrogen peroxide, in which the hydrogen peroxide is prepared in situ using mixtures of O₂/CO or O₂/H₂, in the presence of suitable catalysts. The use of molecular oxygen instead of preformed hydrogen peroxide has obvious economical advantages.

For example in patent JP62-292738, phenol is obtained by the oxidation of benzene in the presence of black Pd, 1,10-phenanthroline, acetic acid and a mixture of oxygen and CO. The reaction is generally carried out at temperatures of about 150-220°C using explosive mixtures of O₂/CO (ratio 1:1). Operating according to this process yields of 2.2% are obtained in 12 hours corresponding to a turnover/hour of palladium of 0.34.

According to patent JP 0142.445, phenol is prepared by the oxidation of benzene in the presence of palladium acetate, 1,10-phenanthroline and a mixture of O₂/CO. The reaction is carried out at a temperature of 180°C, obtaining after 1 hour a conversion of 7.7% corresponding to a turnover of the catalyst equal to 17.

Tatsumi et al. (J. Chem. Soc. Chem. Commun., 1992, 1446-1447) disclose a process for the preparation of phenol from benzene with H₂ and O₂ which uses a catalyst consisting of palladium supported on TS-1. Operating according to this process, a conversion of the benzene of 0.07% is obtained with a turnover of palladium of 13.5.

The term "hourly turnover of palladium", as used in the present description, refers to the efficiency of the catalyst and is calculated as a molar ratio between the phenol produced in 1 hour and the palladium charged.

These processes of the known art, however, do not have a productivity, referring to the quantity of phenol produced, which is sufficiently high for them to be of interest for industrial application. In addition, the catalysts used have a limited activity under the operating conditions.

There is therefore an evident necessity for further improvements in the field of the production of phenol.

It has now been found that it is possible to satisfy these demands of the known art by means of a simple and convenient process which is based on the use of catalytic complexes of palladium with a nitrogenated ligand and a non-coordinating counter-ion for the synthesis of hydrogen peroxide in situ.

The process of the present invention has substantial advantages and in particular:
(i) the possibility of using much less drastic reaction conditions, above all in terms of the temperature;
(ii) the possibility of using mixtures of oxygen or air and non-explosive CO;
(iii) a greater efficiency of the catalyst, which can be advantageously used in reduced quantities;
(iv) higher conversion and selectivity to phenol.

In accordance with this, a first aspect of the present invention relates to a process for the synthesis of phenol from benzene which comprises reacting the benzene with hydrogen peroxide in a biphasic system consisting of an aqueous phase and an organic phase immiscible with this, in the presence of a titanium silicalite, wherein said process is characterized in that the hydrogen peroxide is prepared in situ by the reaction of O₂, CO and water in the presence of a catalytic system deriving from the combination of:
(a) an organic or inorganic salt of palladium;
(b) a mono or polydentate nitrogenated ligand capable of binding itself to the palladium atom; and
(c) a non-coordinating organic acid.

Catalysts which can be used in the process of the present invention Known as titanium silicalites correspond to the general formula (I):

xTiO₂ · (1-x)SiO₂ (I)

wherein: x is between 0.0001 and 0.04.

The above titanium silicalites can be prepared according to the method described in U.S. patent 4.410.501 which also describes their structural characteristics.

Titanium silicalites can also be used in which part of the titanium is substituted by other metals such as boron, aluminum, iron or gallium. These substituted titanium silicalites and the methods for their preparation are described in European patent applications 226.257, 226.258 and 266.825.

These catalysts are used in quantities ranging from 5 to 80 g per litre of reaction mixture, preferably between 10 and 50 g per litre of reaction mixture.

Palladium salts which can be used in the process of the present invention are for example: palladium acetate, palladium perfluoro-octanoate, palladium nitrate and palladium sulfate. Palladium acetate and palladium perfluoro-octanoate are preferred.

The quantity of palladium salt used in the process of the present invention is generally between 0.1 and 10 mmoles per litre of organic phase, preferably between 0.2 and 5 mmoles/litre.

Monodentate nitrogenated ligands suitable for the purposes of the present invention can be nitrogenated heterocyclic compounds such as pyridine and its derivatives such as, for example, alkylpyridines.

Bidentate nitrogenated ligands can be selected from those having general formula (II) 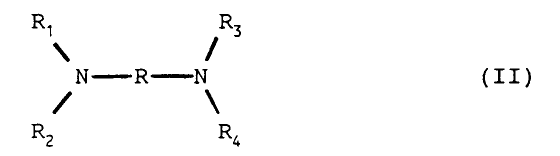 wherein: R represents a C₂-C₄ alkyl radical optionally substituted with an alkyl or aryl group; R₁-R₄, the same or different, each represent a C₁-C₁₀ alkyl radical, a C₃-C₁₀ cycloalkyl radical or a C₆-C₁₂ aromatic radical optionally substituted with a C₁-C₄ alkyl or alkoxyl radical;
or from those having general formula (III): 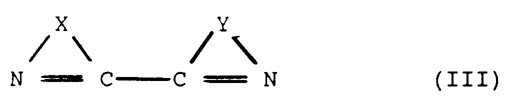 wherein: X and Y, the same or different, represent a bridged organic group having at least three atoms in the bridge of which at least two are carbon atoms. When, as well as the carbon atoms, groups X and Y contain other atoms, these are preferably selected from oxygen and nitrogen.

Examples of polydentate nitrogenated ligands are:
N,N,N',N'-tetramethylethylenediamine-1,2; N,N,N',N'-tetranethyl-propanediamine-1,3; 4,4'-dimethyl-2,2'-bipyridyl; 4,4'-diphenyl-2,2'-bipyridyl; 5,5'-dimethyl-2,2-bipyridyl; 5-methyl-2,2'bipyridyl;4-methyl-1,10-phenanthroline; 5-methyl-1,10-phenanthroline; 4,7-dimethyl-1,10-phenanthroline; 3,8-dimethyl-1,10-phenanthroline; 4,7-diphenyl-1,10-phenanthroline; 4,7-dichloro-1,10-phenanthroline; 3,4,7,8-tetramethyl-1,10-phenanthroline; 4,4'-dimethyl-5,5'-bioxazole; 2,2'-tetrahydro-bioxazole; 2,9-dichloro-1,10-phenanthroline; 2,9-dibutylphenanthroline; 2,9-dimethylphenanthroline; 2,9-dimethyl-4,7-diphenylphenanthroline; 4,7-diphenyl-2,9-dimethylphenanthroline, 2,4,6-tri-(2-pyridyl)-1,3,5-triazine and 6,7-dimethyl-2,3-di-(2-pyridyl)-quinoxaline.

Preferred ligands for the purposes of the present invention are 4,7-diphenyl-2,9-dimethylphenanthroline (bathocuproine), 2,9-dimethyl-phenanthroline (neocuproine).

In the process of the present invention, a molar ratio ligand/palladium ranging from 1:1 to 30:1, preferably from 3:1 to 15:1, is used.

Non-limiting examples of non-coordinating organic acids which can be used in the process of the present invention are selected from perfluoroalkylcarboxylic acids having formula (IV):

CₙF₂ₙ₊₁COOH (IV)

wherein n is between 1 and 15, preferably between 1 and 7 or from perfluoroalkylsulfonic acids having formula (V):

CₙF₂ₙ₊₁SO₃H (V)

wherein n is between 1 and 8, preferably between 1 and 4. Perfluoro-octanoic acid and trifluoroacetic acid are preferred.

Advantageous results are obtained using molar ratios acid/palladium ranging from 1/1 to 100/1, preferably between 10/1 and 80/1.

The reaction medium used is a double phase system consisting of an aqueous phase and an organic phase immiscible with water. The volume ratio between the organic phase and the aqueous phase is between 1/2 and 8/1, preferably between 1/1 and 4/1.

The organic phase consists of benzene and an organic cosolvent belonging to the group of ketones such as for example acetone, methyl-ethylketone and methyl-isobutylketone, nitriles such as acetonitrile or alcohols such as terbutanol.

Preferred co-solvents are ketone compounds in particular methyl-ethylketone and methyl-isobutylketone. The ratio between benzene and cosolvent is between 5/1 and 1/5, preferably between 2/1 and 1/2.

The gaseous phase consists of carbon monoxide and oxygen and is used in the reaction so as to maintain a total pressure of more than 1 atm and preferably between 5 and 100 atm.

The molar ratio CO/O₂ is preferably between 1:99 and 12:88, preferably between 3:97 and 10:90.

According to an embodiment of the process of the present invention, the reaction can be carried out using air instead of pure oxygen.

The hydrogen peroxide produced is immediately used by the titanium silicalite to oxidize the benzene and does not accumulate in the reaction medium.

The reaction is typically carried out at temperatures ranging from 0°C to 100°C and preferably at temperatures ranging from 20° to 80°C.

For example, in a batch process the palladium salt, nitrogenated ligand and acid are introduced in an apparatus into the water/organic solvent mixture and the reaction is carried out at room temperature, under stirring, for a time necessary for guaranteeing the complete formation of the catalytic complex.

After addition of the titanium silicalite, the carbon monoxide and oxygen are then fed at the pressures and in the quantities desired and defined above.

The following examples, which have the sole purpose of describing the present invention in more detail, should in no way be considered as limiting the scope of the invention itself.

### EXAMPLE 1

5 ml of water, 5 ml of benzene and 5 ml of methylisobutylketone are charged into an autoclave equipped with a glass ampule and magnetic stirrer. 0.7 mg (3.1 µmoles) of Pd(OAc)₂, 4.5 mg (12.4 µmoles) of bathocuproine (molar ratio ligand:palladium = 4), 21.2 mg (186 µmoles) of trifluoroacetic acid (molar ratio acid:palladium = 60) are added to this mixture which is left under stirring at 25°C for 4 hours. 400 mg of titanium silicalite (TS1) catalyst are added to the solution thus obtained.

The reactor is then pressurized with 5 atm of carbon monoxide and 65 atm of oxygen (molar ratio CO:O₂ = 7:93). The reaction is carried out under vigorous stirring (600 rpm) at 70°C. After 6 hours of reaction 1.8 mmoles of phenol are obtained, corresponding to:
- a conversion of benzene of 3.2%
- a selectivity to phenol of 91%
- an overall turnover of palladium of 580.

Selectivity refers to the molar selectivity with respect to the converted benzene.

### EXAMPLE 2

The reaction is carried out as described in example 1, but using 5 ml of methyl-ethylketone as cosolvent.

After 6 hours of reaction 1.2 mmoles of phenol are obtained, corresponding to a conversion of benzene of 2.1% and a turnover of palladium of 380. The selectivity with respect to benzene is > 95%.

### EXAMPLE 3

The reaction is carried out as described in example 1, but using 5 ml of acetone as co-solvent.

After 6 hours of reaction 0.93 mmoles of phenol are obtained, corresponding to a conversion of benzene of 1.6% and a turnover of palladium of 300. The selectivity with respect to benzene is > 95%.

### EXAMPLE 4

The reaction is carried out as described in example 1, but using 5 ml of terbutanol as cosolvent.

After 6 hours of reaction 0.9 mmoles of phenol are obtained, corresponding to a conversion of benzene of 1.5% and a turnover of palladium of 290. The selectivity with respect to benzene is > 95%.

### EXAMPLE 5

The reaction is carried out as described in example 1, but using as acid 77.0 mg (186 µmoles) of perfluoro-octanoic acid (molar ratio acid:palladium = 60).

After 6 hours of reaction 1.1 mmoles of phenol are obtained, corresponding to a conversion of benzene of 2 % and a turnover of palladium of 360. The selectivity with respect to benzene is > 95%.

### EXAMPLE 6

The reaction is carried out as described in example 4, but using as ligand 2.6 mg (12.4 µmoles) of neocuproine.

After 6 hours of reaction 1.2 mmoles of phenol are obtained, corresponding to a ccnversion of benzene of 2.1% and a turnover of palladiun of 380. The selectivity with respect to benzene is > 95%.

## Claims

1. A process for the synthesis of phenol by the catalytic oxidation of benzene with hydrogen peroxide in the presence of titanium silicalite, characterized in that the hydrogen peroxide is prepared in situ by the reaction of oxygen, carbon monoxide and water in the presence of catalytic complexes consisting of:
(a) an organic or inorganic salt of palladium;
(b) a mono or polydentate nitrogenated ligand capable of binding itself to the palladium atom; and
(c) a non-coordinating organic acid.

2. The process according to claim 1, characterized in that the titanium silicalite has general formula (I):
xTiO₂ · (1-x)siO₂ (I)
wherein: x is between 0.0001 and 0.04.

3. The process according to claim 2, wherein in the compound having formula (I) part of the titanium is substituted by other metals such as boron, aluminum, iron or gallium.

4. The process according to claim 1, wherein the quantity of titanium silicalite is between 5 and 80 g per litre of reaction mixture.

5. The process according to claim 4, wherein the quantity of titanium silicalite is between 10 and 50 g per litre of reaction mixture.

6. The process according to claim 1, characterized in that the monodentate nitrogenated ligand is a nitrogenated heterocyclic compound selected from pyridine and alkylpyridine.

7. The process according to claim 1, characterized in that the polydentate nitrogenated ligand is selected from compounds having formula (II) 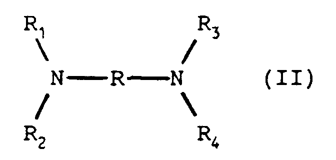 wherein: R represents a C₂-C₄ alkyl radical optionally substituted with an alkyl or aryl group;
R₁-R₄, the same or different, each represent a C₁-C₁₀ alkyl radical, a C₃-C₁₀ cycloalkyl radical or a C₆-C₁₂ aromatic radical optionally substituted with a C₁-C₄ alkyl or alkoxyl radical.

8. The process according to claim 1, characterized in that the nitrogenated ligand is selected from compounds having formula (III): 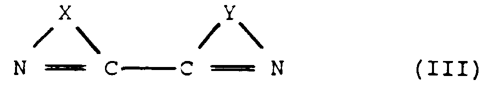 wherein: X and Y, the same or different, represent a bridged organic group having at least three atoms in the bridge of which at least two are carbon atoms.

9. The process according to claim 8, wherein when, as well as the carbon atoms, groups X and Y contain other atoms, these are selected fromm oxygen or nitrogen.

10. The process according to claim 1, characterized in that the polydentate nitrogenated ligand is selected from
N,N,N',N'-tetramethylethylenediamine-1,2; N,N,N',N'-tetramethyl-propanediamine-1,3; 4,4'-dimethyl-2,2'-bipyridyl; 4,4'-diphenyl-2,2'-bipyridyl; 5,5'-dimethyl-2,2-bipyridyl; 5-methyl-2,2'bipyridyl; 4-methyl-1,10-phenanthroline; 5-methyl-1,10-phenanthroline; 4,7-dimethyl-1,10-phenanthroline; 3,8-dimethyl-1,10-phenanthroline; 4,7-diphenyl-1,10-phenanthroline; 4,7-dichloro-1,10-phenanthroline; 3,4,7,8-tetramethyl-1,10-phenanthroline; 4,4'-dimethyl-5,5'-bioxazole; 2,2'-tetrahydrobioxazole; 2,9-dichloro-1,10-phenanthroline; 2,9-dibutylpnenanthroline; 2,9-dimethylphenanthroline; 2,9-dimethyl-4,7-diphenylphenanthroline; 4,7-diphenyl-2,9-dimethylphenanthroline, 2,4,6-tri-(2-pyridyl)-1,3,5-triazine and 6,7-dimethyl-2,3-di-(2-pyridyl)-quinoxaline.

11. The process according to claim 10, characterized in that the ligand is selected from 4,7-diphenyl-2,9-dimethylphenanthroline and 2,9-dimethylphenanthroline.

12. The process according to claim 1, characterized in that the molar ratio ligand:palladium is between 1:1 and 30:1.

13. The process according to claim 12, characterized in that the molar ratio ligand:palladium is between 5:1 and 15:1.

14. The process according to claim 1, characterized in that the palladium salt is selected from palladium acetate, palladium perfluoro-octanoate, palladium nitrate and palladium sulfate.

15. The process according to claim 14, characterized in that the palladium salt is palladium acetate or palladium perfluoro-octanoate.

16. The process according to claim 1, characterized in that the concentration of palladium salt is between 0.1 and 10 mmoles of palladium salt per litre of organic phase.

17. The process according to claim 16, characterized in that the concentration of palladium salt is between 0.2 and 5 mmoles of palladium salt per litre of organic phase.

18. The process according to claim 1, characterized in that the acid is selected from perfluoroalkylcarboxylic acids having formula (IV):
CₙF₂ₙ₊₁COOH (IV)
wherein n is between 1 and 15 or from perfluoroalkylsulfonic acids having formula (V):
CₙF₂ₙ₊₁SO₃H (V)
wherein n is between 1 and 8.

19. The process according to claim 18, characterized in that the perfluoroalkylcarboxylic acids are represented by the formula CₙF₂ₙ₊₁COOH wherein n is between 1 and 7.

20. The process according to claim 18, characterized in that the perfluoroalkylsulfonic acids are represented by the formula CₙF₂ₙ₊₁SO₃H wherein n is between 1 and 4.

21. The process according to claim 18, characterized in that the acid is selected from perfluorooctanoic acid and trifluoroacetic acid.

22. The process according to claim 1, characterized in that the molar ratio acid:palladium is between 1:1 and 100:1.

23. The process according to claim 22, characterized in that the molar ratio acid:palladium is between 10:1 and 80:1.

24. The process according to claim 1, characterized in that the organic phase consists of benzene and a cosolvent selected from ketones, nitriles or alcohols with a volumetric ratio between the two ranging from 5:1 to 1:5.

25. The process according to claim 24, characterized in that the volumetric ratio benzene/co-solvent is between 2/1 and 1/2.

26. The process according to claim 24, characterized in that the cosolvent is selected from acetone, methyl-ethylketone, methyl-isobutylketone, acetonitrile or terbutanol.

27. The process according to claim 1, characterized in that the volumetric ratio between the organic phase and the aqueous phase is between 1:2 and 8:1.

28. The process according to claim 27, characterized in that the volumetric ratio between the organic phase and the aqueous phase is between 1:1 and 4:1.

29. The process according to claim 1, characterized in that the reaction is carried out at a total pressure higher than 1 atm and with a molar ratio carbon monoxide:oxygen ranging from 1:99 to 12:88.

30. The process according to claim 29, characterized in that the pressure is between 5 atm and 100 atm and the molar ratio carbon monoxide:oxygen is between 3:97 and 10:90.

31. The process according to claim 1, wherein the reaction is carried out using air as oxygen source.

32. The process according to claim 1, characterized in that the reaction is carried out at a temperature ranging from 0 to 100°C.

33. The process according to claim 30, wherein the temperature is between 20°C and 80°C.
